# EUROPEAN PATENT SPECIFICATION

(11) **EP 1 446 092 B1**
(45) Date of publication and mention of the grant of the patent: **13.08.2008**
(21) Application number: 02785364.7
(22) Date of filing: 06.11.2002
(51) Int. Cl.: A61K 8/44, A61K 8/64, A61K 8/97, A61Q 5/00, A61Q 19/00

(54) **USE OF CAPRYLOYL GLUTAMATE SALTS AND CAPRYLOYL HYDROLYSATE SALTS OF WHEAT AND/OR RICE PROTEIN IN DETERGENT AND COSMETIC COMPOSITIONS**
VERWENDUNG VON CAPRYLOYLGLUTAMAT UND CAPRYLOYLHYDROLYSAT-SALZEN VON WEIZEN- UND/ODER REISPROTEINEN IN REINIGUNGS- UND KOSMETISCHEN ZUSAMMENSETZUNGEN
UTILISATION DE SELS CAPRYLOYL-GLUTAMATES OU DE SELS DE CAPRYLOYL-HYDROLYSATS DE PROTEINES DE BLE ET/OU DE RIZ DANS DES COMPOSITIONS DETERGENTES OU COSMETIQUES

(30) Priority: 07.11.2001 IT TO20011053
(43) Date of publication of application: 18.08.2004
(73) Proprietor: Zschimmer & Schwarz Italiana S.p.A., 13038 Tricerro (Vercelli) (IT)
(72) Inventor: ARIOTTO, Angelo, I-15030 Terruggia (Alessandria) (IT); GUALA, Fabrizio, I-13039 Trino (Vercelli) (IT); MERLO, Elisabetta, I-13039 Trino (Vercelli) (IT); VILLA, Giovanni, I-20030 Paderno Dugnano (Milano) (IT)
(74) Representative: Rambelli, Paolo
(86) International application number: PCT/EP2002/012369
(87) International publication number: WO 2003/039496

(56) References cited:
- EP-A- 1 074 247
- WO-A-01/99376
- WO-A-02/056840
- WO-A-02/062304
- FR-A- 2 760 746
- US-A- 5 458 881
- PATENT ABSTRACTS OF JAPAN vol. 017, no. 017 (C-1016), 13 January 1993 (1993-01-13) & JP 04 243809 A (KANEBO LTD), 31 August 1992 (1992-08-31)
- PATENT ABSTRACTS OF JAPAN vol. 016, no. 128 (C-0924), 2 April 1992 (1992-04-02) & JP 03 294298 A (SEIWA KASEI:KK), 25 December 1991 (1991-12-25)

## Description

The present invention relates to the use of capryloyl glutamate, capryloyl hydrolysate salts of wheat and/or rice proteins in detergent or cosmetic compositions provided with sebum regulating, anti-dandruff and/or anti-odour properties and simultaneously provided with hydrating and self-preservative properties.

In the formulation of cosmetic or detergent compositions with sebum regulating, anti-dandruff and/or anti-odour properties the use of derivatives of caprylic acid is known. However, the problem with the derivatives commonly utilised is often that of excessively delipidising the corneal layer of the skin by acting as a solvent of the functional lipids contained in it and simultaneously removing the water-soluble substances from it. All this causes excessive drying of the skin and fragility of the hair, which problems are commonly resolved by adding suitable hydrating agents to the formulations. In the formulation of cosmetic or detergent compositions in general it is moreover necessary to add self-preservative agents which have the function of preserving the composition from pollution by bacteria and other micro-organisms such as, for example, fungi and yeasts, which can be the cause of infections in man. However, the disadvantage associated with the use of self-preservative agents is that in order to be efficacious they must often be utilised at irritant concentrations or at concentrations at which they sensitise the tissue with which they come into contact.

The present invention therefore has the object of providing a detergent or cosmetic composition provided with the above indicated functional properties (that is to say, sebum regulating, anti-dandruff and/or anti-odour) which does not have the above-mentioned disadvantages.

This object has been achieved by the present inventors, who have identified various derivatives of caprylic acid which, when formulated in detergent or cosmetic compositions, are, not only able to perform the sebum regulating, anti-dandruff and/or anti-odour activity, but are simultaneously able to confer on the said composition hydrating and self-preservative properties.

These derivatives are salts of capryloyl glutamate and capryloyl hydrolysate of rice and/or wheat protein.

The salts of capryloyl glutamate can be obtained by the Schotten-Baumann reaction by making the amino group of the glutamic acid react with capryloyl chloride in a basic environment, according to the following reaction: 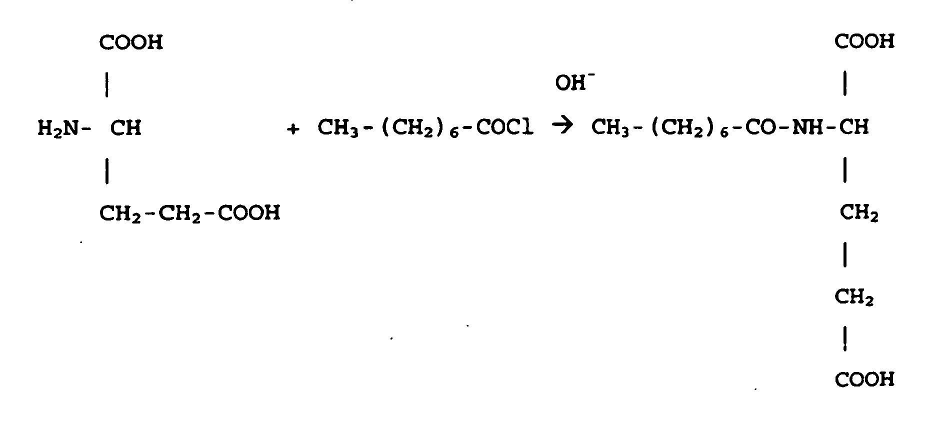 In the same way the salts of capryloyl hydrolysate can be obtained by means of the Schotten-Baumann reaction by making the amino group present in the hydrolysate react with capryloyl chloride in a basic environment according to the following reaction:

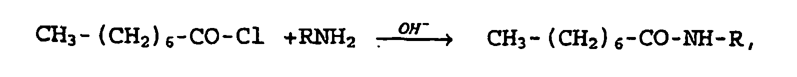

Wherein R is the peptide residue obtainable by means of hydrolysis of wheat and/or rice proteins.

This peptide residue (or hydrolysate) is obtained by acid hydrolysis (with HCl) of gluten (wheat) and/or husks (rice) and subsequent filtration to separate the product obtained from the drogs.

As an alternative to chemical hydrolysis, an enzymatic hydrolysis can also be used, by exploiting the amylase and protease enzymes.

The product obtained from hydrolysis is a mixture of peptides having a molecular weight between 1000 and 4000 Dalton. In the ambit of the present specification this product will sometimes be indicated as "peptide residue" and other times as "hydrolysate of wheat and/or rice protein".

During the Schotten-Baumann reaction the dissociated carboxylic groups present are neutralised by salification with a base chosen in dependence on the desired salt, for example NaOH or KOH. Alternatively, to obtain salts of a weak base such as TEA the sodium or potassium salt is brought to a pH of 2.0-3.0 with phosphoric acid to obtain the acid form and subsequently dissolved in water and neutralised with the desired base.

In the above-described salts the dissociated carboxylic groups are preferably neutralised with cations chosen from groups which consist of: cations belonging to the groups of alkaline metals or other monovalant cations such as Cu⁺; cations belonging to the groups of alkaline earth metals or other bivalent cations such as Pb²⁺; trivalent cations such as Al³⁺; polyvalent cations such as Sn⁴⁺ ; NH₄⁺ or amine bases chosen from triethanol amine, monoethanol amine, diethanol amine, monoisopropanol amine, tri-isopropanol amine, 2-amino butanol, amino ethylpropanediol, arginine, lisine, ornithine, amino methyl propanol, amino methyl propanediol and 2-amino-2-hydroxymethyl-1,3-propanediol. Such neutralising cations can also be utilised in combination with one another.

A subject of the invention is therefore the use of a salt of capryloyl glutamate and/or capryloyl hydrolysate of wheat and/or rice proteins as self-preservative and hydrating agents and simultaneously sebum regulating, anti-dandruff and/or anti-odour agents in the formulation of a detergent or cosmetic composition free from other self-preservative agents and other hydrating agents, or including other self-preservative and/or hydrating agents at a non-efficacious concentration and/or insufficient to confer the desired characteristics of self-preservative and/or hydration in an analogous or similar composition free of the said salts (composition).

The expression "analogous or similar composition free from the said salt" is intended to indicate a composition constituted by the same components in the same quantities as the composition of the invention, but which is differentiated from it by the fact that the capryloyl glutamate salt and/or capryloyl hydrolysate salt of wheat and/or rice proteins is replaced by an equal quantity of other anionic surfactant agent.

In general it can be understood that a detergent or cosmetic composition has the desired self-preservative characteristic when it responds positively to the tests for evaluation of the efficacy of the preserving power. Such tests are conducted according to the indications provided by the United States Pharmacopoeia (USP) and/or by the Cosmetic, Toiletry and Fragrance Association (CTFA). These methods, and other similar methods, involve the inoculation of the samples with micro-organisms of different types and counting the aerobic microbes of these at different times to evaluate the survival level.

The self-preservative efficacy of the salts utilised in the present invention has been confirmed by means of challenge tests conducted on aqueous solutions of capryloyl glutamate in the form of sodium salt, inoculated with mixed inoculants (see example 1).

The tests, as well as confirming the self-preservative efficacy of the composition, also demonstrated that it increases with the reduction of pH. Consequently, it is preferred that the cosmetic or detergent composition has a pH less than or equal to 5, more preferably a pH in the range from 3.5 to 5.0.

The use of the above-indicated salts in the formulation of detergent or cosmetic compositions thus makes it possible to eliminate or in any event substantially reduce the concentration of the usual self-preservative agents the concentration of which, if present, will nevertheless be less than the threshold of irritant and/or sensitising activity.

Among the self-preservative agents commonly utilised in detergent or cosmetic compositions of the prior art, but absent from the composition according to the invention, or at most present in non-self-preservative concentrations are, for example, formaldehyde, chlorine, hypochlorite, compounds which release chlorine, chlorine dioxide, iodine and iodofers, phenol, cresol, chlorocresol, amphoteric compounds with self-preservative characteristics, chlorhexidine, peracetic acid and dehydroacetic acid, mercury compounds, alcohols, sorbic acid, benzoic acid, salicylic acid, boric acid, formic acid, propionic acid and salts derived from the use, bronopol, 5-bromo-5-nitrodioxane, hexamethylene tetramine, DMDM idantoine, various inantoines such as MDM idantoine, chloracetamide, ureas such as imidazolidinyl - urea and diazolidinyl urea, inorganic sulphites, triclosane, parabenes, isothiazolinones, usnic acid and its salts, chlorophene, hexachlorophene, dichlorophene, bromocheorophene, trichlorocarbon, chloroflourocarbon, benzamidines classified as self-preservatives, amines classified as self-preservatives, dimethyloxazolidine, ethylbicyeloxazolidine, dimethylhydroxymethylpyrazole, polyaminopropylbiguamide, sodium hydroxy-methylglycinate, methyldibromoglutaronitrile, glyceryl monolaurate and their mixtures.

The non-self-preservative concentration varies in dependence on the compound considered, and can be easily determined by the man skilled in the art since the above-listed compounds are all known and usually utilised in cosmetics as self-preservatives.

In general it can be understood that a detergent or cosmetic composition has the desired hydrating characteristics when it is capable of obviating the symptoms and manifestations of dry skin and hair (according to the definition of "hydrating compounds" in "A Short Textbook of Cosmetology", K.F. De Polo, first edition 1998).

The salts utilised in the present invention have such hydrating properties.

The molecules of capryloyl glutamate and capryloyl hydrolysate, once they come into contact with the skin, in fact separate into glutamates and hydrolysate on the one hand and fatty acid on the other.

Both wheat and rice hydrolysate protein and glutamic acid have hydrating properties.

In fact, the protein hydrolysate, in contact with the skin, forms a continuous protective layer over the corneal layer thereof, protecting it from external aggressions and increasing the hydration by reduction of TEWL (Trans Epidermal Water Loss). Moreover such protein hydrolysates are sources of amino acid, which constitutes 40% of the natural moisturising factor (NMF). The glutamic acid on the other hand enters into a series of biological cycles contributing to the correct hydration of the skin and to maintenance of the natural cutaneous acidity thanks to the two to carboxylic groups. In fact, it constitutes 15% of the keratins and forms part of the ε (γ-glutamyl) lysine bond present in the in involucrin and keratolin proteins which constitute the cellular membrane of the corneal layer. It can be transformed into PCA, one of the components of the NMF, that is to say a set of water soluble substances responsible for the correct hydration, as well as into proline and hydroxyproline (these two amino acids are fundamental for the synthesis of collagen and elastine). The acylglutamates, moreover, act as selective solvents for the principal component of the sebum, i.e., squalene, therefore leaving unaltered the functional lipids of the corneal layer which control the hydration.

The caprylic acid component on the other hand performs its particular action of controlling the increase of pathogenic micro-organisms, dandruff, body odours, the production of sebum and cutaneous disorders.

The use of salts of capryloyl glutamate and/or capryloyl hydrolysate of wheat and/or rice protein in the formulation of the detergent or cosmetic compositions thus make it possible to eliminate or in any event substantially reduce the addition of hydrating factors, emollients or palliatives for the skin, concentrations of which, if present, will nevertheless be less than the efficacious threshold for hydrating activity.

Among the hydrating agents commonly utilised in detergent or cosmetic compositions of the prior art, but absent from the compositions according to the invention, or at most present in non-efficacious concentrations, are, for example, glycerine, sorbitol, propyleneglycol, polyethylene glycol with molecular weight from 200 to 600, Sorbeth-30, urea, lactic acid and its salts, mucopolysaccharides such as ialuronic acid and chondroitin sulphate, orotic acid, lanolin, petrolatum, mineral oils, occlusive substances which hydrate the skin by preventing the evaporation of water, and their mixtures.

The concentration which is not efficacious for hydration varies in dependence on the compound considered, and can be easily determined by the man skilled in the art since the above-listed compounds are all known and commonly utilised in cosmetics as hydratants.

The detergent or cosmetic compositions preferably include an aqueous medium, although the salts utilised can equally well be used in emulsions.

The total concentration of salts of capryloyl glutamate and/or capryloyl hydrolysate of wheat and/or rice protein in the compositions is preferably one which is sufficient in itself to confer the desired self-preservative characteristics and hydrating action, that is such as to confer the said characteristics in combination with other self-preservative and/or hydrating agents present in concentrations which are not sufficient per se to confer such characteristics.

This concentration lies preferably between 0.5% and 25% by weight of active substance with respect to the total composition, more preferably between 1.5% and 15% by weight of active substance. In this range of concentration, in fact, the salts present are able correctly to perform all their functions.

The detergent or cosmetic compositions can include at least one further and separate surfactant agent chosen from anionic surfactants such as, for example, salts of alkyl sulphates and alkyl ether sulphates (for example sodium, magnesium, TEA, MEA and ammonia salts), salts of amide/amide ether sulphates, salts of alkyl semisulphosuccinates and alkyl sulphosuccinates, salts of alkyl ether semisulphosuccinates and alkyl ether sulphosuccinates, salts of acyl amide semisulphosuccinates and of acyl amide sulphosuccinates, salts of dodecyl benzene sulphonic acid, salts of alkyl/alkyl ether sulphoacetates, salts of sulphonated and/or sulphated organic molecules (for example, α-olefin sulphonates), salts of alkyl/alkyl ether carboxylates, alkyl phosphonates, esters of phosphoric acid, salts of acyl septionates; amphoteric surfactants, such as, for example, alkyl betaine, alkyl amidopropylbetaine, oxides of amine and of amides, amphocarboxyacetates and amphocarboxydiacetates, amphocarboxypropionates and amphocarboxydipropionates; non-ionic surfactants such as, for example, various amides, ethoxylated and non-ethoxylated fatty amines, ethoxylated nonylphenol, APG (alkyl polyglucosides), AEG (alkyl ethoxyglucosides), esters/ethers of fatty acids with glycerol and/or ethoxylated and non-ethoxylated sugars, various ethoxylated/propoxylated and non-ethoxylated/propoxylated esters, ethoxylated/propoxylated and non-ethoxylated/propoxylated fatty alcohols, silicone molecules; cationic surfactants containing, for example, at least one atom of quaternary nitrogen.

Some products in which the compositions can be used are: shampoo, foam baths, foam showers, washing up liquid, liquid soaps, solid soaps, lotions, emulsions of various types, balsams, products having simultaneous detergent and conditioning effects on the skin and/or hair, oils, emollient and detergent milks, face cream and/or body cream and/or hair cream, detergents for intimate hygiene, brilliantine, permanent wave solutions, hair colouring, dentifrice, medicated cosmetics and pharmaceutical products.

The examples which follow are provided solely for the purpose of illustration and or not intended to limit in any way the scope of the invention.

### EXAMPLES

### Example 1: test on the self-preservative efficacy

To evaluate the self-preservative efficacy of sodium salts of capryloyl glutamate a challenge test was conducted on a cosmetic composition comprising:
Sodium lauryl ether sulphate 20E (27% a.m.) = 37%
Cocoamidopropyl betaine (30% a.m.) = 5%
Sodium capryloyl glutamate (about 1.5% a.m.) = 5%
Water = to 100%
pH = 5.

Four mixed inoculations illustrated in the following table 1 (gram+ bacteria; gram- bacteria; yeasts; filamentary fungi) were prepared and each of these was inoculated with a specimen of the above-indicated composition.

**Table 1**

| | | |
|---|---|---|
| GRAM+ BACTERIA: | 1x10⁷ | Staphylococcus aureas ATCC6538 Staphylococcusepidermidis ATCC 12228 |
| GRAM- BACTERIA: | 1x10⁷ | Eschericha coli ATCC 8739 Pseudomonas aeruginosa ATCC 9027 Enterobacter cloacae ATCC 13047 Pseudomonas putida (from cosmetics) |
| YEASTS: | 1x10⁷ | Candida albicans ATCC 10231 Saccharomyces cerevisiae ATCC 9763 |
| FILAMENTARY FUNGI: | 1x10⁵ | Aspergillus niger ATCC 16404 Penicillium funiculosum ATCC 9644 |

The challenge test consists in monitoring the survival of the inoculants over time; this survival is indicative of the self-preservative capacity of the cosmetic product during the period of use by the consumer.

In particular, survival of the inoculants was checked after 24 hours, seven days and 28 days from the inoculation. Monitoring at 24 hours represents a datum for the purpose of evaluating the rapidity of the self-presezvative system, that at 7 days indicates on the other hand the degree of risk of pollution of the product, whilst the test at 28 days, as well as representing confirmation of the previously obtained data, makes it possible to show up the possible formation of resistant strains.

The results obtained are illustrated in the following Table 2.

**Table 2**

| Values obtained in the survival tests of the inoculations expressed in colony forming units per gram of product (c.f.u./g) | | | | |
|---|---|---|---|---|
| Survival | Gram + (c.f.u./g) | Gram - (c.f.u./g) | Filamentary Fungi (c.f.u./g) | Yeasts (c.f.u./g) |
| 24 hours | <10 | 10² | <10 | 10² |
| 7 days | <10 | <10 | <10 | <l0 |
| 28 days | <10 | <10 | <10 | <10 |

By successive dilutions the minimum inhibiting concentration (MIC) of an aqueous solution at pH 5 of sodium capryloyl glutamate against some micro-organisms involved in the etiology of dandruff and some skin and related diseases was calculated. The results obtained are shown in the following Table 3.

**Table 3**

| MICRO-ORGANISM | MIC (%) |
|---|---|
| Pytyrosporum ovale^{a} | 4.5% |
| Staphylococcus aureas^{b} | 1.5% |
| Staphylococcus epidermidis^{b} | 6.0% |
| Propionibactrium acnes^{b} | 5.5% |
| Pseudomonas aeruginosa^{c} | 1.5% |

| | |
|---|---|
| ^{a} agent involved in the etiology of dandruff and in many skin disorders ^{b} agent involved in the etiology of acne ^{c} agent which determines pathological events above all in individuals with compromised immune defences; it is one of the principal protagonists of hospital infections. | |

### Example 2: Hydrating Face Cleaner without self-preservatives

Sodium capryloyl glutamate (30% active material): 6%
Sodium cocoamphodiacetate (26% active material): 10%
Sodium lauryl sulphoacetate (65% active material): 1.5%
PEG-7 glyceril cocoate: 2%
Citric acid: as required to pH 5.0
NaCl: as needed
Colour and perfume: as needed
Water: to 100%

### Example 3: sebum regulating face gel

Sodium capryloyl hydrolysate of rice protein (30% active material): 9%
Laurylmidopropyl betaine (30% active material): 20%
Sodium cocoamphodiacetate (26% active material): 3%
Cocoamidopropylaminooxide (30% active material): 10%
Citric acid: as needed to pH 5.0
NaCl, colour, perfume and water: to 100%

### Example 4: sports shower gel with deodorant effect

Potassium capryloyl hydrolysate of wheat protein (30% active
material): 6%
Laurylethoxysulphate of magnesium ethoxylate 2 moles (25% active material): 15%
Laurylamidopropyl betaine (30% active material): 20%
PEG-7 glyceryl coccoate: 1%
Citric acid: as required to pH 4.5
NaCl, colour, perfume and water: to 100%

### Example 5: intimate detergent

Salts of triethanolamine capryloyl hydrolysate of wheat protein (30% active material): 7%
Sodium capryloyl glutamate (30% active material): 3%
Laurylethoxysulphate of magnesium ethoxylate 2 moles (25% active material): 10%
Cocoamidopropyl betaine (30% active material): 6%
Disodiumlaurylethoxysemisulphosuccinate (30% active material): 4%
Citric acid: as need to pH 3.5 to 4.0
NaCl, colour, perfume and water: to 100%

### Example 6: anti-dandruff shampoo

Sodium capryloyl glutamate (30% active material): 10%
Laurylethoxysulphate of sodium ethoxylate 3 moles (27% active material): 35%
Laurylamidopropyl betaine (30% active material): 5%
Laurylsulphate of ammonia (26% active material): 9%
DEA coccoamide (100% active material): 1%
Citric acid: as needed to pH 4.5
NaCl, colour, perfume and water: to 100%

### Example 7:

Potassium capryloyl hydrolysate of rice protein (30% active material): 5%
Laurylethoxysulphate of sodium ethoxylate 3 moles (27% active material): 30%
Potassium capryloyl glutamate (30% active material): 3%
Laurylsulphate of tri-ethanol amine (39% active material): 6% Citric acid: as needed to pH 4.2
NaCl, colour, perfume and water: to 100%

### Example 8: antidandruff shampoo

Potassium capryloyl glutamate (30% active material): 8%
Lauryamidopropyl betaine (30% active material): 10%.
Laurylethoxysulphate of sodium ethoxylate 3 moles (27% active material): 10%
Sodium lauryl sulphoacetate (65% active material): 1%
Lauryl sulphate of ammonia (26% active material): 10%
Citric acid: as needed to pH 5.0
NaCl, colour, perfume and water: to 100%

### Example 8: hand wash

Sodium capryloyl hydrolysate of wheat protein (30% active material): 8%
Ethoxylated sodium lauryl ethoxysulphate 2 mole (27% active material): 20%
Coccoamidopropylbetaine (30% active material): 4%
Citric acid: as needed to pH 4.0
NaCl, colour, perfume and water: to 100%

## Claims

1. Use of capryloyl glutamate and/or capryloyl hydrolysate salts of wheat and/or rice proteins as self-preservative and hydrating agents and simultaneously as sebum regulating, anti-dandruff and/or anti-odour agents in a detergent or cosmetic composition which is free from other self-preservative agents and other hydrating agents, or which comprises other self-presenrative and/or hydrating agents in a non-efficacious and/or insufficient concentration to confer the desired self-preservative and/or hydration characteristics in an analogous or similar composition free from the said salts.

2. Use according to Claim 1, wherein the said salt is selected from the group consisting of : salts with alkaline metal cations or other monovalent cations such as Cu⁺; salts with alkaline earth metal cations or other bivalent cations such as Pb²⁺; salts with trivalent cations such as Al³⁺; salts with polyvalent cations such as Sn⁴⁺; salts with NH₄⁺ or with an amino base selected from tri-ethanol amine, monethanol amine, diethanol amine, monoisopropanol amine, tri-isopropanol amine, 2-amino butanol, amino ethyl propanediol, arginine, lisine, ornithine, amino methyl propanol, amino methyl propanediol, 2-amino-2-hydroxymethyl-1,3-propanediol; and their combinations.

3. Use according to Claim 1 or 2, wherein the said detergent or cosmetic composition further includes an aqueous. medium.

4. Use according to any of Claims 1 to 3, wherein the said detergent or cosmetic composition has a pH value less than or equal to 5.

5. Use according to any of Claims 1 to 4, wherein the said further hydrating agent is selected from the group consisting of: glycerine, sorbitol, propylene glycol, polyethylene glycol with molecular weight from 200 to 600, Sorbeth-30, urea, lactic acid and its salts, mucopolysaccharides such as ialuronic acid and chondroitin sulphate, orotic acid, lanolin, petrolatum, mineral oils, occlusive substances which hydrate the skin by preventing the evaporation of water, and their mixtures.

6. Use according to any of Claims 1 to 5, wherein the said further self-preservative agent is selected from the group consisting of: formaldehyde, chlorine hypochlorite, compounds releasing chlorine, bioxide of chlorine, iodine and iodophers, phenol, cresol, chlorocresol, amphoteric compounds with self-preservative characteristics, chlorhexidine, peracetic acid and dehydroacetic acid, compounds of mercury, alcohols, sorbic acid, benzoic acid, salicylic acid, boric acid, formic acid, propionic acid and salts derived from these, bronopol, 5-bromo-5-nitrodioxane, hexamethylene tetramine, DMDM idantoine, various idantoines such as MDM idantoine, chloracetamide, ureas such as imidazolidinyl urea and diazodinyl urea, inorganic sulphites, trichlosane, parabenes, isothiazolinones, usnic acid and its salts, chlorophene, hexachlorophene, dichlorophene, bromochlorophene, trichlorocarbon, chlorofluorocarbon, benzamidines classified as self-preservatives, amines classified as self-preservatives, dimethyloxazolidine, ethylbicycloxazolidine, dimethyl hydroxymethyl pirazol, polyaminopropyl biguanide, sodium hydroxymethylglycinate, methyldibromoglutaronitrile, glyceryl monolaurate and their mixtures.

7. Use according to any of Claims 1 to 6, wherein the said detergent or cosmetic composition includes a further and separate surfactant agent selected from anionic, cationic, non-ionic, amphoteric surfactants and their mixtures.

8. Use according to Claim 7, wherein the said further and separate surfactant agent is selected from sodium laurylsulphate, sodium laurylethoxysulphate and their mixtures.

9. Use according to any of Claims 1 to 8, wherein the concentration of the said salt in the said detergent or cosmetic composition is in the range from 0.5 to 25% by weight of active material with respect to the total weight of the composition.

10. Use according to Claim 9, wherein the said concentration is in the range of 1.5 to 15% by weight of active material.

## Patentansprüche

1. Verwendung von Capryloylglutamat-Salzen und/oder Capryloylhydrolysat-Salzen von Weizen- und/oder Reisproteinen als Konservierungsmittel und Hydratisiermittel und gleichzeitig als talgregulierende, schuppenhemmende und/oder geruchshemmende Mittel in einem Reinigungsmittel oder in einem kosmetischen Präparat, die keine anderen Konservierungsmittel und keine anderen Hydratisiermittel enthalten, oder die andere Konservierungsmittel und/oder Hydratisiermittel in einer unwirksamen Konzentration und/oder in einer Konzentration enthalten, die unzureichend ist, um die gewünschten konservierenden Eigenschaften und/oder hydratisierenden Eigenschaften in einer analogen oder einer ähnlichen Zusammensetzung zu vermitteln, die frei von diesen Salzen ist.

2. Verwendung gemäß Anspruch 1, wobei das Salz aus einer Gruppe ausgewählt wird, die besteht aus: Salzen mit Alkalimetall-Kationen oder anderen einwertigen Kationen, wie etwa Cu⁺; Salzen mit Erdalkalimetall-Kationen oder anderen zweiwertigen Kationen, wie etwa Pb²⁺; Salzen mit dreiwertigen Kationen, wie etwa Al³⁺; Salzen mit mehrwertigen Kationen, wie etwa Sn⁴⁺; Salzen mit NH₄⁺ oder mit einer Aminobase, die aus Triethanolamin, Monoethanolamin, Diethanolamin, Monoisopropanolamin, Triisopropanolamin, 2-Aminobutanol, Aminoethylpropandiol, Arginin, Lysin, Ornithin, Aminomethylpropanol, Aminomethylpropandiol, 2-Amino-2-hydroxymethyl-1,3-propandiol; sowie deren Kombinationen ausgewählt werden.

3. Verwendung gemäß Anspruch 1 oder 2, wobei das Reinigungsmittel oder das kosmetische Präparat weiters ein wässriges Medium enthalten.

4. Verwendung gemäß irgendeinem der Ansprüche 1 bis 3, wobei das Reinigungsmittel oder das kosmetische Präparat einen pH-Wert von weniger oder gleich 5 besitzt.

5. Verwendung gemäß irgendeinem der Ansprüche 1 bis 4, wobei das weitere Hydratisiermittel aus einer Gruppe ausgewählt wird, die besteht aus: Glyzerin, Sorbit, Propylenglykol, Polyethylenglykol mit einem Molekulargewicht von 200 bis 600, Sorbeth-30, Harnstoff, Milchsäure und ihre Salze, Mucopolysaccharide, wie etwa Hyaluronsäure und Chondroitinsulfat, Orotsäure, Lanolin, Vaseline, Mineralöle, okklusive Substanzen, die die Haut **dadurch** hydratisieren, dass sie die Verdunstung von Wasser verhindern, sowie deren Gemische.

6. Verwendung gemäß irgendeinem der Ansprüche 1 bis 5, wobei das weitere Konservierungsmittel aus einer Gruppe ausgewählt wird, die besteht aus: Formaldehyd, Chlor, Hypochlorit, Verbindungen, die Chlor freisetzen, Chlordioxid, Jod und Jodophore, Phenol, Kresol, Chlorkresol, amphotere Verbindungen mit konservierenden Eigenschaften, Chlorhexidin, Peroxyessigsäure und Dehydroessigsäure, Verbindungen von Quecksilber, Alkohole, Sorbinsäure, Salizylsäure, Borsäure, Ameisensäure, Propionsäure und die daraus erhaltenen Salze, Bronopol, 5-Brom-5-nitrodixan, Hexamethylentetramin, DMDM-Hydantoin, verschiedene Hydantoine, wie etwa MDM-Hydantoin, Chloracetamid, Harnstoffe, wie etwa ImidazolidinylHarnstoff und Diazodinyl-Harnstoff, anorganische Sulfite, Triclosan, Parabene, Isothiazolinone, Usninsäure und ihre Salze, Chlorophen, Hexachlorophen, Dichlorophen, Bromchlorophen, Trichlorkohlenstoff, Chlorfluorkohlenstoff, Benzamidine klassifiziert als Konservierungsmittel, Amine klassifiziert als Konservierungsmittel, Dimethyloxazolidin, Ethylbicyclooxazolidin, Dimethylhydroxymethylpyrazol, Polyaminopropylbiguanid, Natriumhydroxymethylglycinat, Methyldibromglutaronitril, Glycerilmonolaurat sowie deren Gemische.

7. Verwendung gemäß irgendeinem der Ansprüche 1 bis 6, wobei das Reinigungsmittel oder das kosmetische Präparat ein weiteres und getrenntes oberflächenaktives Mittel aufweisen, das aus anionischen, kationischen, nichtionischen, amphoteren oberflächenaktiven Mitteln sowie deren Gemischen ausgewählt wird.

8. Verwendung gemäß Anspruch 7, wobei das weitere und getrennte oberflächenaktive Mittel aus Natriumlaurylsulfat, Natriumlaurylethoxysulfat und deren Gemischen ausgewählt wird.

9. Verwendung gemäß irgendeinem der Ansprüche 1 bis 8, wobei die Konzentration des Salzes im Reinigungsmittel oder im kosmetischen Präparat im Bereich von 0,5 bis 25 Gewichtsprozenten der aktiven Substanz bezogen auf das Gesamtgewicht der Zusammensetzung liegt.

10. Verwendung gemäß Anspruch 9, wobei die Konzentration im Bereich von 1,5 bis 15 Gewichtsprozenten der aktiven Substanz liegt.

## Revendications

1. Utilisation de sels capryloyl-glutamates et/ou de sels de capryloyl-hydrolysats de protéines de blé et/ou de riz comme agents auto-conservateurs et hydratants, et en même temps comme agents sébo-régulateurs, anti-pellicules et/ou anti-odeur, dans une composition détergente ou cosmétique qui ne contient pas d'autres agents auto-conservateurs ni d'autres agents hydratants, ou qui contient d'autres agents auto-conservateurs et/ou d'autres agents hydratants, mais en une concentration sans effets et/ou insuffisante pour conférer les caractéristiques d'auto-conservation et/ou d'hydratation voulues à une composition analogue ou similaire ne contenant pas desdits sels.

2. Utilisation conforme à la revendication 1, pour laquelle ledit sel est choisi dans l'ensemble constitué par les sels formés avec les cations de métaux alcalins ou d'autres cations monovalents tels que Cu⁺, les sels formés avec les cations de métaux alcalino-terreux ou d'autres cations bivalents tels que Pb²⁺, les sels formés avec des cations trivalents tels que Al³⁺, les sels formés avec des cations polyvalents tels que Sn⁴⁺, et les sels formés avec le cation ammonium NH₄⁺ ou avec une base aminée choisie parmi les triéthanolamine, monoéthanolamine, diéthanolamine, monoisopropanolamine, triisopropanolamine, 2-aminobutanol, amino-éthyl-propanediol, arginine, lysine, ornithine, amino-méthyl-propanol, amino-méthyl-propanediol et 2-amino-2-hydroxyméthyl-1,3-propanediol, ainsi que les combinaisons de tels sels.

3. Utilisation conforme à la revendication 1 ou 2, ladite composition détergente ou cosmétique comprenant en outre un milieu aqueux.

4. Utilisation conforme à l'une des revendications 1 à 3, ladite composition détergente ou cosmétique présentant un pH inférieur ou égal à 5.

5. Utilisation conforme à l'une des revendications 1 à 4, pour laquelle ledit autre agent hydratant est choisi dans l'ensemble contitué par les suivants : glycérol, sorbitol, propylèneglycol, polyéthylèneglycol présentant une masse molaire de 200 à 600, Sorbeth-30, urée, acide lactique et ses sels, mucopolysaccharides comme l'acide hyaluronique et le chondroïtine-sulfate, acide orotique, lanoline, pétrolatum, huiles minérales, et substances occlusives qui hydratent la peau en empêchant l'évaporation de l'eau, ainsi que les mélanges de ces corps.

6. Utilisation conforme à l'une des revendications 1 à 5, pour laquelle ledit autre agent auto-conservateur est choisi dans l'ensemble contitué par les suivants : formaldéhyde, chlore, hypochlorite, composés libérant du chlore, dioxyde de chlore, iode et composés iodés, phénol, crésol, chloro-crésol, composés amphotères possédant des propriétés auto-conservatrices, chlorhexidine, acide peracétique, acide déhydracétique, composés du mercure, alcools, acide sorbique, acide benzoïque, acide salicylique, acide borique, acide formique, acide propionique, sels dérivés de ces acides, bronopol, 5-bromo-5-nitro-dioxane, hexaméthylène-tétramine, DMDM-hydantoïne, divers dérivés d'hydantoïne tels que la MDM-hydantoïne, chloracétamide, dérivés de l'urée comme l'imidazolidinyl-urée et la diazolidinyl-urée, sulfites inorganiques, triclosan, parabens, isothiazolinones, acide usnique et ses sels, chlorophène, hexachlorophène, dichlorophène, bromochlorophène, triclocarban, chlorofluorocarbures, benzamidines classées comme auto-conservateurs, amines classées comme auto-conservateurs, diméthyloxazolidine, éthyl-bicyclooxazolidine, diméthyl-hydroxyméthyl-pyrazole, polyaminopropyl-biguanide, hydroxyméthyl-glycinate de sodium, méthyl-dibromoglutaronitrile et monolaurate de glycéryle, ainsi que les mélanges de ces composés.

7. Utilisation conforme à l'une des revendications 1 à 6, ladite composition détergente ou cosmétique comprenant un agent tensioactif distinct supplémentaire, choisi parmi les agents tensioactifs anioniques, cationiques, non-ioniques et amphotères et leurs mélanges.

8. Utilisation conforme à la revendication 7, pour laquelle ledit agent tensioactif distinct supplémentaire est choisi parmi du laurylsulfate de sodium, du lauryl-éthoxy-sulfate de sodium et leurs mélanges.

9. Utilisation conforme à l'une des revendications 1 à 8, pour laquelle la concentration dudit sel dans ladite composition détergente ou cosmétique se situe dans l'intervalle allant de 0,5 à 25 %, en poids de substance active rapporté au poids total de la composition.

10. Utilisation conforme à la revendication 9, pour laquelle ladite concentration se situe dans l'intervalle allant de 1,5 à 15 % en poids de substance active.
